# EUROPÄISCHE PATENTSCHRIFT

(11) **EP 1 164 996 B1**
(45) Veröffentlichungstag und Bekanntmachung des Hinweises auf die Patenterteilung: **22.10.2003**
(21) Anmeldenummer: 00916981.4
(22) Anmeldetag: 21.03.2000
(51) Int. Cl.: A61F 13/02, A61K 9/70, A61K 31/4406, C11B 9/00

(54) **NIKOTIN-TTS MIT EINEM ZUSATZ VON MONOTERPENKETONEN**
TRANSDERMAL THERAPEUTIC SYSTEM WITH NICOTINE AND ADDITION OF MONOTERPENE KETONES
SYSTEMES THERAPEUTIQUES TRANSDERMIQUES CONTENANT DE LA NICOTINE, AVEC AJOUT DE MONOTERPENE-CETONES

(30) Priorität: 26.03.1999 DE 19913732
(43) Veröffentlichungstag der Anmeldung: 02.01.2002
(73) Patentinhaber: LTS Lohmann Therapie-Systeme AG, 56626 Andernach (DE)
(72) Erfinder: BRACHT, Stefan, D-56299 Ochtendung (DE)
(74) Vertreter: Flaccus, Rolf-Dieter, Dr.
(86) Internationale Anmeldenummer: EP0002457
(87) Internationale Veröffentlichungsnummer: WO00057824

(56) Entgegenhaltungen:
- US-A- 4 933 184
- US-A- 5 362 496
- US-A- 5 599 554
- US-A- 5 820 877

## Beschreibung

Die vorliegende Erfindung betrifft den Zusatz geruchsverbessernder Stoffe zu transdermalen therapeutischen Systemen (TTS) mit einem Gehalt an Nikotin. Sie betrifft insbesondere nikotinhaltige TTS, welche derartige Zusätze enthalten, sowie Verfahren zum Überdecken des unangenehmen Geruchs solcher TTS, sowie die Verwendung geruchsverbessernder Stoffe zur Überdeckung des durch den Nikotingehalt bedingten unangenehmen Geruchs solcher TTS.

Nikotinhaltige TTS finden weltweit breite Verwendung bei der Raucherentwöhnung. Die im Markt befindlichen Systeme weisen allerdings einen deutlichen Nikotingeruch auf, der im besonderen bei der Entnahme aus der Verpackung und der Applikation der Systeme wahrgenommen wird.

Im Rahmen der Haltbarkeitszeit von typischerweise 2 bis 3 Jahren kann es durch partielle Zersetzung zur deutlichen Verstärkung dieses Geruches und Veränderung hin zu subjektiv sehr unangenehmen Geruchsnoten kommen.

In der WO 95/08324 A1 ist ein Verfahren zur Herstellung von mindestens zweischichtigen TTS unter Verwendung eines leicht flüchtigen Inhaltsstoffes als ausschließlichem Lösungsmittel beschrieben worden. Diese TTS können verschiedene Wirkstoffe, darunter auch Nikotin, sowie u. a. Menthol oder andere flüchtige Terpenderivate als Hautpenetrations-Enhancer enthalten. Eine besondere Wirkung dieser Zusatzstoffe bei nikotinhaltigen TTS hinsichtlich des Geruchs wurde nicht beschrieben. Hinsichtlich der "flüchtigen Terpenderivate" wird nicht differenziert zwischen Monoterpenalkoholen und Monoterpenketonen.

Die EP 0 356 382 A2 offenbart TTS auf der Basis von bestimmten Block-Copolymeren, wobei auch Nikotin als Wirkstoff enthalten sein kann. Zur Verbesserung der Hautpenetration werden Eucalyptol bzw. Eukalyptusöle vorgeschlagen. Dabei wird vor allem Cineol als Hauptbestandteil hervorgehoben; Inhaltsstoffe von Minzenölen werden nicht in Betracht gezogen. Der Aspekt des unangenehmen Geruchs von nikotinhaltigen Pflastern bleibt unberücksichtigt.

Die US 5 599 554 A betrifft die transmucosale oder transdermale Applikation von Nikotin, wobei die verwendeten Zusammensetzungen auch Duft- oder Geschmacksstoffe enthalten können. Der charakteristische Geruch von Nicotin wird zwar erwähnt, aber nicht als nachteilig beschrieben. Aromatische Verbindungen wie Menthol oder Eucalyptol, nicht aber ätherische Minzenöle oder Terpenketone werden als optionale Zutaten genannt. Über die Funktion dieser Zusätze werden keine Angaben gemacht. Vermutlich dienen sie der Geschmacksverbesserung bei oralen Darreichungsformen.

Die US 5 593 684 beschreibt eine Behandlungsmethode, welche auf der gleichzeitigen transmucosalen und transdermalen Verabreichung von Nikotin beruht. Dabei werden terpenhaltige Pflanzensekrete als "etherische Öle" in Pastillen zur oralen Applikation verwendet, um den unangenehmen Geschmack des Nicotins zu überlagern.

Die US 4 933 184 A betrifft TTS mit verbesserter transdermaler Wirkstoffabgabe, u. a. auch für Nikotin, wobei Menthol als Enhancer eingesetzt wird; andere in ätherischen Ölen von Minzenarten vorkommende Substanzen, z. B. Monoterpenketone, werden nicht erwähnt. Ein Minzöl wurde als Enhancer alternativ zu Menthol geprüft, lieferte aber diesen Effekt überraschenderweise nicht. Im übrigen bezieht sich diese Druckschrift lediglich auf die Verbesserung der Wirkstoffpermeation, und nicht auf ein Verfahren zur Verbesserung des Geruchs von TTS.

Der Erfindung liegt daher die Aufgabe zugrunde, bei nikotinhaltigen TTS gemäß Oberbegriff des Anspruchs 1 diesen Eigengeruch durch Zusatz von geeigneten Riechstoffen zu neutralisieren bzw. angenehm zu überdecken.

Die Lösung der Aufgabe wurde nun im Zusatz von ätherischen Ölen verschiedener Minzenarten bzw. deren Inhaltsstoffen, insbesondere Monoterpenketonen, gefunden. Erfindungsgemäß können diese Zusätze verwendet werden, um den unangenehmen Geruch nikotinhaltiger TTS zu überdecken oder zu verbessern. Die erfindungsgemäßen TTS weisen einen Gehalt an mindestens einem aus einer Minzenart gewonnenen ätherischen Öl, das überwiegend Monoterpenketone enthält enthält, oder einem Monoterpenketon, das in diesen ätherischen Ölen vorkommt, auf.

Die Inhaltsstoffe der ätherischen Öle verschiedener Minzenarten werden dominiert von Produkten des Terpenstoffwechsels, genauer von Monoterpenen.

Minzenöle zeichnen sich allgemein durch ihren angenehmen, erfrischenden Geruch aus. Verwendung finden beispielsweise Pfefferminzöl, Krausminzöl oder Poleiminöl, die jeweils aus verschiedenen Pflanzen gewonnen werden. Die charakteristischen Monoterpene in diesen Ölen lassen sich in Monoterpenalkohole und Monoterpenketone unterteilen.
Typische Monoterpenalkohole sind Menthol, Isomenthol, Neomenthol, Neoisomenthol und Isopulegol.
Typische Monoterpenketone sind Menthon, Isomenthon, Carvon, Piperiton, Pulegon und Isopulegon.
Praktisch alle diese Vertreter existieren als Enantiomere sowohl in einer optisch links- als auch rechtsdrehenden Form.

Stellvertretend für diese Gruppe wurden die ätherischen Öle der Pfefferminze (Oleum Menthae piperitae), der Krauseminze (Oleum Menthae crispae) und der (japanischen) Minze (Oleum Menthae arvensis) untersucht.
Pfefferminzöl und insbesondere Minzöl werden von Monoterpenalkoholen, speziell Menthol dominiert. Krauseminzöl enthält dagegen überwiegend Monoterpenketone, speziell Carvon (vgl. Monographie "Pfefferminzöl" im Europäischen Arzneibuch 1997; Monographie "Minzöl" im Deutschen Arzneibuch 1997, sowie G. Schneider: Pharmazeutische Biologie, 2. Auflage 1988, BI Wissenschaftsverlag, S. 342-345).
Als Einzelstoffe wurden (-)-Menthol und (-)-Menthon als typischer Monoterpenalkohol bzw. typisches Monoterpenketon getestet.

### Beispiele:

Zur Überprüfung der Wirkung solcher Zusätze wurde ein vereinfachtes Riechmodell entwickelt.
Nikotin wurde in einer Konzentration von 7 Gew.% mit Miglyol 812 vermischt. Miglyol 812 ist ein gesättigtes Triglycerid, das als geruchsneutraler Träger dient. Die Konzentration von 7 Gew.% Nikotin entspricht etwa der in TTS eingesetzten Wirkstoffkonzentration von 5-10 Gew.%. Es ergibt sich für das Nikotin in Miglyol ein mit TTS vergleichbarer Dampfdruck und damit eine ähnliche Geruchsintensität.
Dieser Testmischung wurden folgende 5 Teststoffe bzw. -gemische zugesetzt:
(-)-Menthol, (-)-Menthon, Pfefferminzöl (Qualität gem. Europäisches Arzneibuch), Krauseminzöl (Qualität gem. Deutscher Arzneimittel Codex DAC) und Minzöl (Qualität gem. Deutsches Arzneibuch).

Die zugesetzten Mengen betrugen jeweils 0,5, 1,0 und 2,0 Gew.%.

Daraus ergaben sich 15 Testmuster. Zusätzlich wurde eine Probe ohne geruchsverbessernden Zusatz hergestellt.

Diese 16 Muster wurden von 6 Testpersonen geruchlich beurteilt, wobei den Testpersonen die Art und Menge des jeweiligen Zusatzstoffes nicht bekanntgegeben wurden.

Die Beurteilungskriterien und Bewertungsziffern umfaßten:
1. Nikotin-Geruch: nicht wahrnehmbar (4); schwach (3); mäßig (2); deutlich (1)
2. Gesamteindruck: unangenehm (1); neutral (2); angenehm (3); wohlriechend (4)

Die Bewertung des Gesamteindrucks wurde zur stärkeren Gewichtung gegenüber dem Nikotin-Geruch mit dem Faktor 2 multipliziert, bevor die beiden Werte für jede Probe und Person addiert wurden. Höhere Werte bedeuten eine günstigere Beurteilung.
Aus den so erhaltenen Bewertungsziffern wurde der Mittelwert gebildet.
Der theoretische Minimalwert beträgt 3,0 und der theoretische Maximalwert 12,0.

Das Ergebnis zeigt Tabelle 1:

| Testprodukt/Menge | 0,5 Gew.% | 1,0 Gew.% | 2,0 Gew.% |
|---|---|---|---|
| (-)-Menthol | 4,0 | 4,1 | 4,9 |
| (-)-Menthon | 6,6 | 6,4 | 8,6 |
| Pfefferminzöl | 6,9 | 8,7 | 9,3 |
| Krauseminzöl | 7,3 | 7,7 | 9,6 |
| Minzöl | 6,0 | 7,0 | 9,0 |

Das Produkt ohne Zusatz erzielte den Wert 4,0.

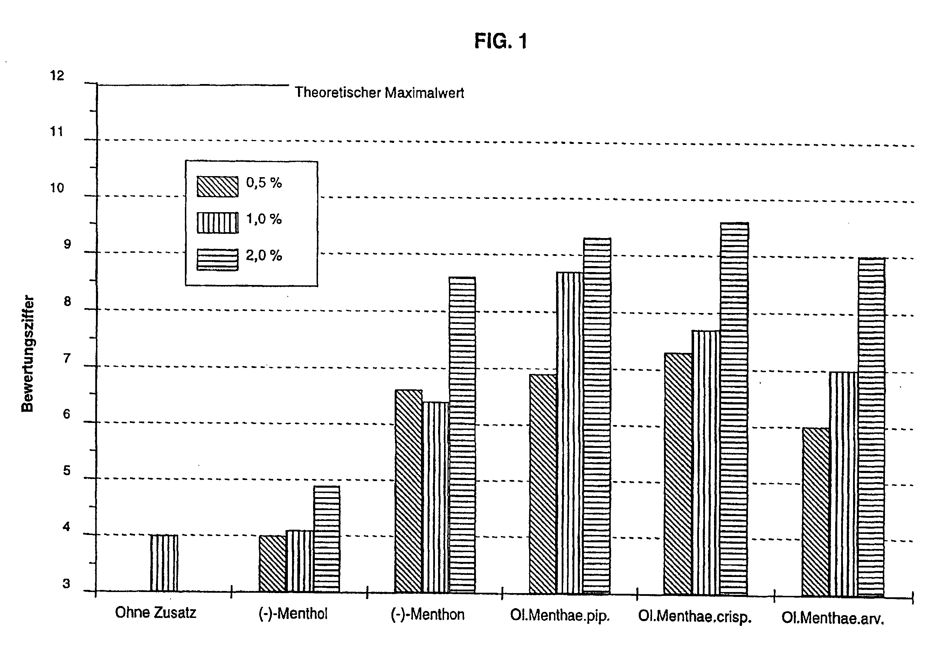

Die graphische Auftragung der Ergebnisse zeigt FIG.1.

Daraus ergibt sich ein sehr überraschend deutlicher Vorteil für Menthon gegenüber Menthol. Das schlechtere Abschneiden des von Menthol dominierten Minzöls (G. Schneider; Pharmazeutische Biologie, 2. Auflage 1988, BI Wissenschaftsverlag, S. 345) gegenüber dem typischerweise bis zu 32 % Menthon enthaltenden Pfefferminzöl (Europäisches Arzneibuch 1997) unterstützt diesen Befund.

Das von Carvon dominierte und praktisch mentholfreie Krauseminzöl erzielt schließlich die beste Bewertung.

In der Summe ergibt sich ein deutlicher Vorteil für Monoterpenketone, bzw. Gemische von Monoterpenalkoholen und Monoterpenketonen gegenüber reinem Monoterpenalkohol.

Die praktische Ausführung des Zusatzes erfindungsgemäßer Stoffe zu nikotinhaltigen TTS stößt wegen deren Leichtflüchtigkeit auf gewisse Schwierigkeiten, die sich aber durch Befolgung der Lehre der PCT/WO 95/08324 ausräumen lassen.
Der Gehalt an Monoterpenketon(en) bzw. an ätherischem Öl in der nikotinhaltigen Matrix der erfindungsgemäßen geruchsverbesserten TTS beträgt 0,1 bis 5,0 Gew.%, vorzugsweise 0,5 bis 2 Gew.%.

Somit stellt der Zusatz erfindungsgemäßer Stoffe zu nikotinhaltigen TTS ein brauchbares Mittel dar, deren unangenehmen Geruch zu verbessern.

Die erfindungsgemäßen TTS mit den im Oberbegriff des Anspruchs 1 genannten Merkmalen sind, wie vorstehend beschrieben, durch einen Gehalt an mindestens einem aus einer Minzenart gewonnenen ätherischen Öl oder einem Monoterpenketon, das in diesen ätherischen Ölen vorkommt, gekennzeichnet.
Vorzugsweise handelt es sich bei dem Monoterpenketon um ein solches aus der Gruppe von Carvon, Dihydrocarvon, Menthon, Isopulegon, Isomenthon, Neomenthon, Neoisomenthon oder Piperiton. Die Monoterpenketone können als reine Enantiomere oder deren Gemische eingesetzt werden.
Als ätherisches Öl wird Krauseminzöl (Oleum Menthae crispae) besonders bevorzugt eingesetzt.
Der Gehalt an Monoterpenketon(en) bzw. an ätherischen Ölen in der nikotinhaltigen Matrix beträgt vorzugsweise 0,1 bis 5,0 Gew.%, besonders bevorzugt 0,5 bis 2 Gew.%.

Die Erfindung betrifft ferner ein Verfahren zum Überdecken eines durch einen Gehalt an Nikotin bedingten unangenehmen Geruchs eines transdermalen therapeutischen Systems, wobei dieses Verfahren dadurch gekennzeichnet ist, daß dem nikotinhaltigen transdermalen therapeutischen System mindestens ein geruchsverbessernder Stoff zugesetzt wird, wobei dieser Stoff ein aus einer Minzenart gewonnenes ätherisches Öl ist oder ein Monoterpenketon, das in einem aus einer Minzenart gewonnenen ätherischen Öl enthalten ist.
Als Monoterpenketone bzw. als ätherisches Öl können dabei vorzugsweise die oben genannten Monoterpenketone bzw. Krauseminzöl eingesetzt werden, wobei die Monoterpenketone als reine Enantiomere oder deren Gemische eingesetzt werden können.

Dabei werden das/die Monoterpenketon(e) bzw. das/die ätherische(n) Öl(e) der nikotinhaltigen Matrix vorzugsweise in einer Konzentration von 0,1 bis 5,0 Gew.%, besonders bevorzugt in einer Konzentration von 0,5 bis 2 Gew.% zugesetzt.

Ferner umfaßt die Erfindung die Verwendung eines aus einer Minzenart gewonnenen ätherischen Öls und/oder eines Monoterpenketons, das in einem aus einer Minzenart gewonnenen ätherischen Öl enthalten ist, zum Überdecken eines unangenehmen Geruchs eines transdermalen therapeutischen Systems, welcher auf einen Gehalt an Nikotin in diesem transdermalen therapeutischen System zurückzuführen ist.

Vorzugsweise handelt es sich bei dem verwendeten Monoterpenketon um ein solches aus der Gruppe von Carvon, Dihydrocarvon, Menthon, Isopulegon, Isomenthon, Neomenthon, Neoisomenthon oder Piperiton, wobei die Monoterpenketone als reine Enantiomere oder deren Gemische eingesetzt werden können.
Als ätherisches Öl wird Krauseminzöl (Oleum Menthae crispae) besonders bevorzugt verwendet.
Bei der erfindungsgemäßen Verwendung zum Überdecken eines unangenehmen Geruchs eines nikotinhaltigen transdermalen therapeutischen Systems wird das/die Monoterpenketon(e) bzw. das ätherische Öl der nikotinhaltigen Matrix vorzugsweise in einer Konzentration von 0,1 bis 5,0 Gew.%, besonders bevorzugt in einer Konzentration von 0,5 bis 2 Gew.%, zugesetzt.

## Patentansprüche

1. Transdermales therapeutisches System mit einer Rückschicht, mindestens einer nikotinhaltigen Schicht oder Zone, die auch haftklebende Eigenschaften aufweisen kann, sowie einer wiederablösbaren Schutzschicht, **gekennzeichnet durch** einen Gehalt an mindestens eine aus einer Minzenart gewonnenen ätherischen Öl, das überwiegend Monoterpenketone enthält, oder einem Monoterpenketon, das in diesen ätherischen Ölen vorkommt.

2. Transdermales therapeutisches System nach Anspruch 1, **dadurch gekennzeichnet, daß** es sich bei dem Monoterpenketon um ein solches aus der Gruppe von Carvon, Dihydrocarvon, Menthon, Isopulegon, Isomenthon, Neomenthon, Neoisomenthon oder Piperiton handelt.

3. Transdermales therapeutisches System nach Anspruch 2, **dadurch gekennzeichnet, daß** die Monoterpenketone als reine Enantiomere oder deren Gemische eingesetzt werden.

4. Transdermales therapeutisches System nach Anspruch 1, **dadurch gekennzeichnet, daß** es sich bei dem ätherischen Öl um Krauseminzöl (Oleum Menthae crispae) handelt.

5. Transdermales therapeutisches System nach einem oder mehreren der vorangehenden Ansprüche, **dadurch gekennzeichnet, daß** der Gehalt an Monoterpenketon(en) in der nikotinhaltigen Matrix 0,1 bis 5,0 Gew.% beträgt, vorzugsweise 0,5 bis 2 Gew.%.

6. Verfahren zum Überdecken eines durch einen Gehalt an Nikotin bedingten unangenehmen Geruchs eines transdermalen therapeutischen Systems, **dadurch gekennzeichnet, daß** dem nikotinhaltigen transdermalen therapeutischen System mindestens ei geruchsverbessernder Stoff zugesetzt wird, wobei dieser Stoff ein aus einer Minzenart gewonnenes ätherisches Öl ist, das überwiegend Monoterpenketone enthält, oder ein Monoterpenketon, das in einem aus einer Minzenart gewonnenen ätherischen Öl enthalten ist.

7. Verfahren nach Anspruch 6, **dadurch gekennzeichnet, daß** daß es sich bei dem ätherischen Öl um Krauseminzöl (Oleum Menthae crispae) handelt.

8. Verfahren nach Anspruch 6, **dadurch gekennzeichnet, daß** es sich bei dem Monoterpenketon um ein solches aus der Gruppe von Carvon, Dihydrocarvon, Menthon, Isopulegon, Isomenthon, Neomenthon, Neoisomenthon oder Piperiton handelt.

9. Verfahren nach Anspruch 8, **dadurch gekennzeichnet, daß** das/die Monoterpenketon(e) vorzugsweise in einer Konzentration von 0,1 bis 5,0 Gew.%, besonders bevorzugt in einer Konzentration von 0,5 bis 2 Gew.%, der nikotinhaltigen Matrix zugesetzt werden.

10. Verwendung eines aus einer Minzenart gewonnenen ätherischen Öls, das überwiegend Monoterpenketone enthält, und/oder eines Monoterpenketons, das in einem aus einer Minzenart gewonnenen ätherischen Öl enthalten ist, zum Überdecken eines unangenehmen Geruchs eines transdermalen therapeutischen Systems, welcher auf einen Gehalt an Nikotin in diesem transdermalen therapeutischen System zurückzuführen ist.

11. Verwendung nach Anspruch 10, **dadurch gekennzeichnet, daß** es sich bei dem Monoterpenketon um ein solches aus der Gruppe von Carvon, Dihydrocarvon, Menthon, Isopulegon, Isomenthon, Neomenthon, Neoisomenthon oder Piperiton handelt.

12. Verwendung nach Anspruch 11, **dadurch gekennzeichnet, daß** das/die Monoterpenketon(e) vorzugsweise in einer Konzentratior von 0,1 bis 5,0 Gew.%, besonders bevorzugt in einer Konzentration von 0,5 bis 2 Gew.%, der nikotinhaltigen Matrix zugesetzt werden.

13. Verwendung nach Anspruch 10, **dadurch gekennzeichnet, daß** es sich bei dem ätherischen Öl um Krauseminzöl (Oleum Menthae crispae) handelt.

## Claims

1. Transdermal therapeutic system having a backing layer, at least one nicotine-containing layer ore zone, which may have pressure-sensitive adhesive properties, as well as a removable protective layer, **characterized by** a content of at least one essential oil extracted from a mint species and containing a predominant portion of monoterpene ketones, or by a content of at least one monoterpene ketone contained in these essential oils.

2. Transdermal therapeutic system according to claim 1, **characterized in that** the monoterpene ketone is one from the group of carvone, dihydrocarvone, menthone, isopulegone, isomenthone, neomenthone, neoisomenthone or piperitone.

3. Transdermal therapeutic system according to claim 2, **characterized in that** the monoterpene ketones are used as pure enantiomers or mixtures thereof.

4. Transdermal therapeutic system according to claim 1, **characterized in that** the essential oil is spearmint oil (Oleum Menthae crispae).

5. Transdermal therapeutic system according to one or more of the preceding claims, **characterized in that** the content of monoterpene ketone(s) in the nicotine-containing matrix is 0.1 to 5.0%-wt., preferably 0.5 to 2%-wt.

6. Process for masking an unpleasant smell, caused by a content of nicotine, of a transdermal therapeutic system, **characterized in that** at least one odour-improving substance is added to the nicotine-containing transdermal therapeutic system, said substance being an essential oil extracted from a mint species and containing a predominant portion of monoterpene ketones, or being a monoterpene ketone contained in an essential oil extracted from a mint species.

7. Process according to claim 6, **characterized in that** the essential oil is spearmint oil (Oleum Menthae crispae).

8. Process according to claim 6, **characterized in that** the monoterpene ketone is one from the group of carvone, dihydrocarvone, menthone, isopulegone, isomenthone, neomenthone, neoisomenthone or piperitone.

9. Process according to claim 8, **characterized in that** the monoterpene ketone(s) are added to the nicotine-containing matrix preferably in a concentration of 0.1 to 5.0%-wt, particularly preferred in a concentration of 0.5 to 2%-wt.

10. Use of an essential oil extracted from a mint species and containing a predominant portion of monoterpene ketones, and/or of a monoterpene ketone contained in an essential oil extracted from a mint species, for masking an unpleasant smell of a transdermal therapeutic system, said smell being caused by a content of nicotine in said transdermal therapeutic system.

11. Use according to claim 10, **characterized in that** the monoterpene ketone is one from the group of carvone, dihydrocarvone, menthone, isopulegone, isomenthone, neomenthone, neoisomenthone or piperitone.

12. Use according to claim 11, **characterized in that** the monoterpene ketone(s) are added to the nicotine-containing matrix preferably in a concentration of 0.1 to 5.0%-wt, particularly preferred in a concentration of 0.5 to 2%-wt.

13. Use according to claim 10, **characterized in that** the essential oil is spearmint oil (Oleum Menthae crispae).

## Revendications

1. Système thérapeutique transdermique pourvu d'une couche postérieure, d'au moins une couche ou zone contenant de la nicotine, pouvant également présenter des propriétés adhésives, ainsi que d'une couche de protection amovible, **caractérisé** en ce qu'il contient au moins une huile essentielle extraite d'une variété de menthe, qui contient principalement des monoterpènecétones ou une monoterpènecétone que l'on trouve dans ce type d'huile essentielle.

2. Système thérapeutique transdermique selon la revendication 1, **caractérisé en ce que** la monoterpènecétone utilisée est un élément choisi dans le groupe des carvones, des dihydrocarvones, des menthones, des isopulégones, des isomenthones, des néomenthones, des néoisomenthones ou des pipéritones.

3. Système thérapeutique transdermique selon la revendication 2, **caractérisé en ce que** la monoterpènecétone est utilisée en tant qu'énantiomère pur ou composé.

4. Système thérapeutique transdermique selon la revendication 1, **caractérisé en ce que** l'huile essentielle utilisée est de l'essence de menthe crépue (Oleum Menthae crispae).

5. Système thérapeutique transdermique selon une ou plusieurs des revendications précédentes, **caractérisé en ce que** le contenu de monoterpènecétone(s) dans la matrice contenant la nicotine s'élève entre 0,1 et 5,0 % en poids, et particulièrement de préférence entre 0,5 et 2 % en poids.

6. Procédé pour masquer l'odeur éventuelle désagréable due au contenu de nicotine d'un système thérapeutique transdermique, **caractérisé** en ce qu'est ajouté au système thérapeutique transdermique contenant de la nicotine au moins une matière destinée à améliorer l'odeur et en ce que cette matière est une huile essentielle extraite d'une variété de menthe qui contient principalement des monoterpènecétones ou une monoterpènecétone contenue dans une huile essentielle extraite d'une variété de menthe.

7. Procédé selon la revendication 6, **caractérisé en ce que** l'huile essentielle utilisée est de l'essence de menthe crépue (Oleum Menthae crispae).

8. Procédé selon la revendication 6, **caractérisé en ce que** la monoterpènecétone utilisée est un élément choisi dans le groupe des carvones, des dihydrocarvones, des menthones, des isopulégones, des isomenthones, des néomenthones, des néoisomenthones ou des pipéritones.

9. Procédé selon la revendication 8, **caractérisé en ce que** la ou les monoterpènecétone(s) utilisée(s) l'est/le sont de préférence dans une concentration de 0,1 à 5,0 % en poids, et particulièrement de préférence dans une concentration de 0,5 à 2 % en poids dans la matrice contenant la nicotine.

10. Utilisation d'une huile essentielle extraite d'une variété de menthe qui contient principalement des monoterpènecétones et/ou une monoterpènecétone contenue dans une huile essentielle extraite d'une variété de menthe pour couvrir l'odeur désagréable d'un système thérapeutique transdermique due au contenu en nicotine de ce système thérapeutique transdermique.

11. Utilisation selon la revendication 10, **caractérisée en ce que** la monoterpènecétone utilisée est un élément choisi dans le groupe des carvones, des dihydrocarvones, des menthones, des isopulégones, des isomenthones, des néomenthones, des néoisomenthones ou des pipéritones.

12. Utilisation selon la revendication 11, **caractérisée en ce que** la ou les monoterpènecétone(s) utilisée(s) l'est/le sont de préférence dans une concentration de 0,1 à 5,0 % en poids, et particulièrement de préférence dans une concentration de 0,5 à 2 % en poids dans la matrice contenant la nicotine.

13. Utilisation selon la revendication 10, **caractérisée en ce que** l'huile essentielle utilisée est de l'essence de menthe crépue (Oleum Menthae crispae).
